# EUROPEAN PATENT SPECIFICATION

(11) **EP 1 086 682 B1**
(45) Date of publication and mention of the grant of the patent: **16.04.2003**
(21) Application number: 99919290.9
(22) Date of filing: 18.05.1999
(51) Int. Cl.: A61K 31/415, A61P 25/00, A61P 43/00

(54) **UTILIZATION OF ARYL(OR HETEROARYL)AZOLYLCARBINOL DERIVATIVES IN THE PREPARATION OF A MEDICAMENT FOR THE TREATMENT OF NEUROGENIC INFLAMMATION**
VERWENDUNG VON ARYL(ODER HETEROARYL)-AZOLYLCARBINOLDERIVATEN IN DER HERSTELLUNG VON MEDIKAMENTEN ZUR BEHANDLUNG VON NEUROGENEN ENTZÜNDUNGEN
UTILISATION DE DERIVES D'ARYL(OU HETEROARYL)AZOLYLCARBINOLS DANS L'ELABORATION D'UN MEDICAMENT POUR LE TRAITEMENT DE L'INFLAMMATION NEUROGENE

(30) Priority: 18.05.1998 ES 9801021
(43) Date of publication of application: 28.03.2001
(73) Proprietor: LABORATORIOS DEL DR. ESTEVE, S.A., 08041 Barcelona (ES)
(72) Inventor: MERCE-VIDAL, Ramon, E-08041 Barcelona (ES); FRIGOLA-CONSTANSA, Jordi, E-08041 Barcelona (ES)
(74) Representative: Carpintero Lopez, Francisco
(86) International application number: ES9900142
(87) International publication number: WO99059524

(56) References cited:
- EP-A- 0 289 380
- MATTIOLI F. AND GUIA M.: 'W-Dialkylamino alkyl Ethers of Phenyl-(5-substituted 1-phenyl-1H-pyrazol-4-yl) methanols with Analgesic and Anti-inflammatory Activity' J. HETEROCYCLIC CHEM. vol. 34, 1997, pages 963 - 968
- HUESO-RODRIGUEZ J.A. ET AL.: 'Preparation of the enantiomers of the analgesic E-3710' BIOORGANIC & MEDICINAL CHEMISTRY LETTERS vol. 3, no. 2, 1993, pages 269 - 272

## Description

The present invention relates to the use of some derivatives of aryl(or heteroaryl)azolylcarbinols of general formula (I), as well as their physiologically acceptable salts, in the manufacture of medicaments, useful in human and/or veterinary therapy, for the treatment of neurogenic inflammation present in different processes such as: diabetes, asthma, cystitis, gingivitis, migraine, dermatitis, rhinitis, psoriasis, inflammation of sciatic and lumbar nerves, gastrointestinal processes, and ocular inflammation.

### Background of the Invention

Neurogenic inflammation is present in very different pathologic conditions, and is characterised by vasodilatation and plasmatic extravasation in zones where non-myelinated fibres can be found. The activation of these fibres frees the mediating substances P and calcitonin gene related peptide (CGRP) from the afferent nerve terminals provoking the characteristic symptoms of neurogenic inflammation [GYORFI, A. et al., J. Clin. Periodontol. 19(10): 731-736 (1992)]. The substance P plays an important role as the main mediator in the induction of neurogenic inflammation by means of an increase in the vascular permeability due to the liberation of histamine from mast cells and by interaction with the endothelial cells [INOUE, H. et al., Inflamm. Res. 44: 125-130 (1995); PIOTROWSKI, W. et al., Br. J. Dermatol. 114: 37-46 (1986); LOWMAN, M.A. et al., Br. J. Pharmacol. 95: 121-130 (1988); FEWTRELL, C.M.S. et al., J. Physiol. 330: 393-411 (1982); STEPHENSON, J. A. et al., Eur. J. Pharmacol. 124: 377-378 (1986); ZICHE, M. et al., Microvas. Res. 40: 264-278 (1990)]. It has also been reported that CGRP can promote the plasmatic extravasation induced by substance P in rat trachea and therefore plays an important role in the modulation of neurogenic inflammation in the respiratory tract [BROKAW, J.J. et al., Lung 170(2): 85-93 (1992)]. The neuropeptides (CGRP and substance P, among others) are found in the primary non-myelinated afferent neurones as neurotransmitters [HOLZER, P., Neuroscience 24: 739-768 (1988)]. Neurogenic inflammation, with reflex vasodilatation of the axon, plasmatic and edema extravasation, can be produced by neuropeptides by means of the activation of primary afferent neurones. In fact, the cutaneous inflammatory responses, including the formation of edema, vasodilatation and wheals, are evoked as response to intradermic injection of neuropeptides [GAMSE, R. et al., Eur. J. Pharmacol. 114: 61-66 (1985); FULLER, R.W. et al., Br. J. Pharmacol. 92: 781-788 (1987)]. Furthermore, nueropeptides have been discovered in lesions of skin diseases and it is suggested that they play a key role in cutaneous inflammation [FARBER, E.M. et al., J. Am. Acad. Dermatol. 14: 305-311 (1986); WALLENGREN, J. et al., Acta Derm. Veneorol. (Stockh.) 66: 23-28 (1986)].

In our patents EP 289380 and ES 9800793 we have described carbinol derivatives of general formula (I) 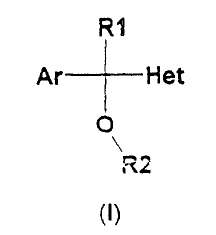 wherein Ar represents a benzene ring or a substituted or unsubstituted thiophene ring, R1 represents a hydrogen atom or a lower alkyl group of C₁ to C₄; R2 represents a dialkylaminoalkyl or azaheterocyclylalkyl radical and Het represents an azol. as well as their physiologically acceptable salts.

In our patents PCT/EP96/05596, ES 9701538, ES 9701728 and ES 9800793 we have also described different procedures for the preparation of enantiomerically pure compounds of general formula (I).

We have now discovered that the compounds of general formula (I), as well as their physiologically acceptable salts, are particularly useful for the manufacture of medicaments, useful in human and/or veterinary therapy, for curing or alleviating neurogenic inflammation present in different processes such as: diabetes, asthma, cystitis, gingivitis, migraine, dermatitis, rhinitis, psoriasis, inflammation of sciatic and lumbar nerves, gastrointestinal processes, and ocular inflammation.

### Detailed Description of the Invention

The present invention relates to the use of derivatives of aryl(or heteroaryl)azolylcarbinols of general formula (I) 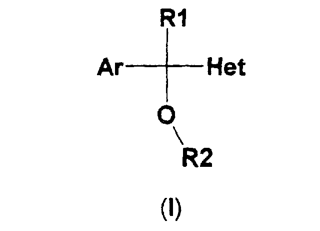 wherein
Ar is an unsubstituted phenyl radical or thienyl radical, or optionally subsituted by 1, 2 or 3 identical or different substituents, selected from the group formed by fluorine, chlorine, bromine, methyl, trifluoromethyl and methoxy;
R1 is a hydrogen atom or an alkyl group C₁ - C₄;
R2 is a dialkyl radical (C₁ - C₄)aminoalkyl (C₂ - C₃), or azaheterocyclyl-alkyl (C₂ - C₃); and
Het is a five membered azotic heterocyclic aromatic ring that contains from one to three nitrogen atoms, unsubstituted or optionally substituted by 1 or 2 substituents, identical or different, selected from the group made up of fluorine, chlorine, bromine and methyl;
or of one of its physiologically acceptable salts,
in the manufacture of a medicament for the treatment of neurogenic inflammation in mammals, including man.

The term "alkyl group C₁ - C₄" represents a straight or branched chain radical that is derived from a saturated hydrocarbon of 1 to 4 carbon atoms, such as methyl, ethyl, propyl, isopropyl, butyl, isobutyl, sec-butyl and *terc*-butyl.

The term dialkyl (C₁ - C₄) aminoalkyl (C₂- C₃) or azaheterocyclylalkyl (C₂ - C₃) represents an alkyl radical of two or three carbon atoms joined to a dialkyl (C₁ - C₄)amine or to a cyclic amine, such as dimethylamino- ethyl, dimethylaminopropyl, diethylaminoethyl, piperidiethyl, morpholinylpropyl, pyrrolidinialkyl, etc.

Illustrative examples of compounds covered by the present invention include:
(±)-5-{α-[2-(dimethylamino)ethoxy]benzyl}-1-methyl-1 *H*-pyrazol,
(±)-5-{α-[2-(dimethylamino)ethoxy]benzyl}-1-methyl-1*H*-pyrazol citrate,
(+)-5-{α-[2-(dimethylamino)ethoxy]benzyl}-1-methyl-1*H*-pyrazol,
(-)-5-{α-(2-(dimethylamino)ethoxy]benzyl}-1-methyl-1*H*-pyrazol,
(+)-5-{α-[2-(dimethylamino)ethoxy]benzyl}-1-methyl-1*H*-pyrazol citrate,
(-)-5-{α-[2-(dimethylamino)ethoxy]benzyl}-1-methyl-1*H*-pyrazol citrate,
(±)-5-{α-[2-(dimethylamino)ethoxy]-2-thienylmethyl}-1-methyl-1*H*-pyrazol,
(±)-5-{α-[2-(dimethylamino)ethoxy]-2-thienylmethyl}-1-methyl-1*H*-pyrazol citrate,
(+)-5-{α-[2-(dimethylamino)ethoxy]-2-thienylmethyl}-1-methyl-1H-pyrazol,
(-)-5-{α-[2-(dimethylamino)ethoxy]-2-thienylmethyl}-1-methyl-1*H*-pyrazol,
(+)-5-{α-[2-(dimethylamino)ethoxy]-2-thienylmethyl}-1-methyl-1*H*-pyrazol citrate, and
(-)-5-{α-[2-(dimethylamino)ethoxy]-2-thienylmethyl}-1-methyl-1*H*-pyrazol citrate.

The compounds of general formula (I) can be synthesised according to the processes described in patents EP 289380 or ES 9800793. The compounds of general formula (I) have a stereogenic centre and the invention relates both to the use of a pure enantiomer and to a mixture of enantiomers. The enantiomers can be prepared by some of the processes described in our patents PCT/EP96/05596, ES 9701538, ES 9701728 or ES 9800793.

Pharmaceutical compositions that contain compounds of general formula (I) are described in our patent applications EP 289380 and ES 9800793.

In the present invention activity of the compounds of general formula (i) against neurogenic inflammation has been demonstrated, studying directly the effect of these compounds on neurogenic inflammation. This study was carried out by determining the inhibition of edema of the ear induced by capsaicine in mice. The effect on the spinal liberation of substance P and CGRP has also been determined in rat. Finally, the effect on spinal liberation of CGRP in polyarthritic rats has been studied.

In the following examples some properties object of the invention for the (±)-5-{α-[2-(dimethylamino)ethoxy]benzyl}-1-methyl-1*H*-pyrazol citrate of the following formula are indicated.

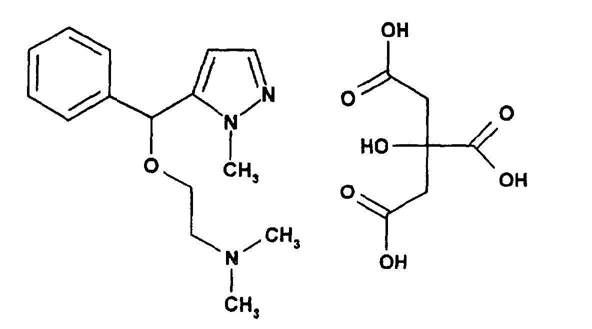

The examples that are indicated below, given by way of illustration, describe some pharmacological trials.

### EXAMPLE 1

### Effect on the mouse ear edema induced by capsaicine.

The mouse ear edema induced by capsaicine has been described as being particularly related to the release of substance P [INOUE, H. et al., Inflamm. Res. 44: 125-130 (1995)]. It has also been reported that capsaicine applied to human skin provokes the response known as neurogenic inflammation. It is supposed that neuropeptides (substance P, CGRP), released from the terminals of afferent C fibres, and histamine, recently released from mast cells, participate in this reaction.

The induction of edema of mouse ear is based on the method of Inoue et al.[ INOUE, H. et al., Br. J. Pharmacol. 110: 1614-1620 (1993)]. In this method capsaicine is applied (250 µg/5 µl of ethanol) to both dorsal and ventral surfaces of the right ear of each mouse (males ICR, 35-45 g). Thirty minutes after applying capsaicine, the animals were sacrificed by cervical dislocation. A disc of 7 mm diameter was extracted from the tissue of the right ear, using a metal disc-cutter and the weight of these discs of tissue was determined. The product, (±)-5-{α-[2-(dimethylamino)ethoxy] benzyl}-1-methyl-1*H*-pyrazol citrate, had been administered orally 1 hour before applying the capsaicine. The results obtained with the said product are summarised below. The anti-inflammatory activity of the said product in this process of neurogenic inflammation is evident.

Effect of (±)-5-{α-[2-(dimethylamino)ethoxy] benzyl}-1-methyl-1*H*-pyrazol citrate on the mouse ear edema induced by capsaicine.

| Compound dosage (mg/kg, orally) | % Inhibition of edema with respect to control group |
|---|---|
| 92 | 32% |
| 184 | 56% |

### EXAMPLE 2

### Effect on the spinal liberation of SP and CGRP in rats

The study was carried out *in vivo*, in rats anaesthetised with halothane. The trial consisted of intrathecal perfusion with an artificial cerebrospinal fluid (ACSF), with a view to collecting the peptides released from the surface layers of the spinal cord while the product under study was being administered locally or systematically. The method used was that described by Collin, E. and co-workers. (Naunyn-Schmiedeberg's Arch. Pharmacol. 349: 387-393, 1994).

The activity of the (±)-5-{α-[2-(dimethylamino)ethoxy]-benzyl}-1-methyl-1*H*-pyrazol citrate, administered intrathecally in the perfusion liquid, at a concentration of 1 µM was studied. As is summarised below, the (±)-5-{α-[2-(dimethylamino)ethoxy]-benzyl}-1-methyl-1*H*-pyrazol citrate inhibited the release both of substance P (SP) and of CGRP. The systematic administration of 46 mg/kg intraperitoneally (i.p.) of the (±)-5-{α-[2-(dimethylamino)ethoxy]-benzyl}-1-methyl-1*H*-pyrazol citrate also reduced the release of substance P and of CGRP.

Effect of the (±)-5-{α-[2-(dimethylamino)ethoxy]-benzyl}-1-methyl-1*H*-pyrazol citrate on the intrathecal release of substance P and CGRP in rat.

| Treatment with (±)-5-{α-[2-(dimethylamino)ethoxy]-benzyl}-1-methyl-1*H*-pyrazol citrate | % Inhibition of intrathecal release with respect to control | |
|---|---|---|
| | Substance P | CGRP |
| 1 µM, intrathecal | -58 % | -60% |
| 46 mg/kg, i.p. | -50% | -60% |

It is of note that the effect of the systematic administration of (±)-5-{α-[2-(dimethylamino)ethoxy]-benzyl}-1-methyl-1*H*-pyrazol citrate on the intrathecal release of substance P lasted 2 hours, the average inhibition being 50% during this time period.

### EXAMPLE 3

### Effect on the spinal release of CGRP in polyarthritic rats.

Polyarthritis was induced by intradermic injection of 0.05 ml of complete Freund adyuvant (*Mycobacterium butiricum* suspended in mineral oil), at about 3 cm from the tip of the tail of Sprague-Dawley rats (weighing 150-175 g). The animals were used four weeks later, when the paws and tail had their maximum inflammation. The rest of the trial was carried out using the same procedure of intrathecal perfusion described in Example 2.

The (±)-5-{α-[2-(dimethylamino)ethoxy]-benzyl}-1-methyl-1*H*-pyrazol citratel was administered interperitoneally at a dosage of 46 mg/kg and the release of intrathecal CGRP was monitored for 3 hours. The comparison with the control group showed that the release of CGRP was much greater in the group treated with (±)-5-{α-[2-(dimethylamino)ethoxy]-benzyl}-1-methyl-1*H*-pyrazol citrate . The measurement of the area under the curve, used as an estimation of the CGRP release during the 3 hours after the treatment, showed an inhibition of 50%. It is therefore clear that (±)-5-{α-[2-(dimethylamino)ethoxy]-benzyl}-1-methyl-1*H*-pyrazol citrate has a notable effect on the release of CGRP, also in polyarthritic rats.

## Claims

1. Use of a derivative of aryl(or heteroaryl)azolylcarbinol of general formula (I) 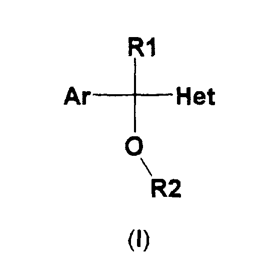 wherein
Ar is a phenyl or thienyl radical, unsubstituted or optionally substituted by 1, 2 or 3 identical or different substituents, selected from the group made up of fluorine, chlorine, bromine, methyl, trifluoromethyl and methoxy;
R1 is a hydrogen atom or an alkyl group C₁ - C₄;
R2 is a dialkyl (C₁ - C₄)aminoalkyl (C₂ - C₃), or azaheterocyclyl-alkyl (C₂ - C₃); and
Het is a five membered azotic heterocyclic aromatic ring that contains from one to three nitrogen atoms, unsubstituted or optionally substituted by 1 or 2 substituents, identical or different, selected from the group made up of fluorine, chlorine, bromine and methyl;
or of one of its physiologically acceptable salts,
in the manufacture of a medicament for the treatment of neurogenic inflammation in mammals, including man.

2. Use, according to claim 1, of a compound of general formula (I) in which R1 is selected from a hydrogen atom or from a group made up of methyl, ethyl, propyl, isopropyl, butyl, isobutyl, *sec*-butyl and *terc*-butyl, in the manufacture of a medicament for the treatment of neurogenic inflammation in mammals, including man.

3. Use, according to claim 1, of a compound of general formula (I) in which R2 is selected from a group made up of dimethylaminoethyl, dimethylaminopropyl, diethylaminoethyl, piperidylethyl, morpholinylpropyl and pyrrolidinylethyl, in the manufacture of a medicament for the treatment of neurogenic inflammation in mammals, including man.

4. Use, according to claim 1, of a compound of general formula (I) selected from the group made up of:
(±)-5-{α-[2-(dimethylamino)ethoxy]benzyl}-1-methyl-1*H*-pyrazol,
(±)-5-{α-[2-(dimethylamino)ethoxy]benzyl}-1-methyl-1*H*-pyrazol citrate,
(+)-5-{α-[2-(dimethylamino)ethoxy]benzyl}-1-methyl-1*H*-pyrazol,
(-)-5-{α-[2-(dimethylamino)ethoxy]benzyl}-1-methyl-1*H*-pyrazol,
(+)-5-{α-[2-(dimethylamino)ethoxy]benzyl}-1-methyl-1*H*-pyrazol citrate,
(-)-5-{α-[2-(dimethylamino)ethoxy]benzyl}-1-methyl-1*H*-pyrazol citrate
(±)-5-{α-[2-(dimethylamino)ethoxy]-2-thienylmethyl}-1-methyl-1*H*-pyrazol,
(±)-5-{α-[2-(dimethylamino)ethoxy]-2-thienylmethyl}-1-methyl-1*H*-pyrazol citrate,
(+)-5-{α-[2-(dimethylamino)ethoxy]-2-thienylmethyl}-1-methyl-1*H*-pyrazol,
(-)-5-{α-[2-(dimethylamino)ethoxy]-2-thienylmethyl}-1-methyl-1*H*-pyrazol,
(+)-5-{α-[2-(dimethylamino)ethoxy]-2-thienylmethyl}-1-methyl-1*H*-pyrazol citrate, and
(-)-5-{a-[2-(dimethylamino)ethoxy]-2-thienylmethyl}-1-methyl-1*H*-pyrazol citrate,
in the manufacture of a medicament for the treatment of neurogenic inflammation in mammals, including man.

## Patentansprüche

1. Verwendung eines Derivats von Aryl(oder Heteroaryl)azolylcarbinols der allgemeinen Formel (I) 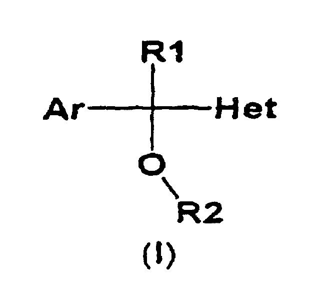 in der Ar für ein Phenylradikal oder ein Thienylradikal steht, die nicht substituiert sind oder nach Wahl durch 1, 2 oder 3 gleiche oder verschiedene Substituenten ausgewählt aus der Gruppe Fluor, Chlor, Brom, Methyl, Trifluormethyl und Methoxy substituiert sind; R1 für ein Wasserstoffatom oder eine C₁-C₄-Alkylgruppe steht; R2 für ein (C₁-C₄) Dialkyl(C₂-C₃) aminoalkylradikal steht, oder für ein Azaheterocyclyl-(C₂-C₃)alkyl steht; und Het für einen fünfgliedrigen, aromatischen, stickstoffhaltigen Heterocyclus steht, mit ein bis drei Stickstoffatomen, der nicht substituiert ist oder nach Wahl durch 1 oder 2 gleiche oder verschiedene Substituenten ausgewählt aus der Gruppe Fluor, Chlor, Brom und Methyl substituiert ist; oder eines seiner physiologisch unbedenklichen Salze, bei der Herstellung eines Arzneimittels für die Behandlung neurogener Entzündungen bei Säugetieren, einschliesslich beim Menschen.

2. Verwendung nach Anspruch 1 von einer Verbindung der allgemeinen Formel (I), in der R1 ausgewählt wird zwischen einem Wasserstoffatom oder der Gruppe bestehend aus Methyl, Ethyl, Propyl, Isopropyl, Butyl, Isobutyl, sec. Butyl und tert. Butyl bei der Herstellung eines Arzneimittels für die Behandlung neurogener Entzündungen bei Säugetieren, einschliesslich beim Menschen.

3. Verwendung nach Anspruch 1 von einer Verbindung der allgemeinen Formel (I), in der R2 ausgewählt wird aus der Gruppe bestehend aus Dimethylaminoethyl, Dimethylaminopropyl, Diethylaminoethyl, Piperidilethyl, Morpholinylpropyl und Pyrrolidinylethyl bei der Herstellung eines Arzneimittels für die Behandlung neurogener Entzündungen bei Säugetieren, einschliesslich beim Menschen.

4. Verwendung nach Anspruch 1 von einer Verbindung der allgemeinen Formel (I), ausgewählt aus der Gruppe bestehend aus:
(±)-5-α-[2-(Dimethylamino)ethoxy]benzyl)-1-methyl-1*H*pyrazol;
(±)-5-α-[2-(Dimethylamino)ethoxy]benzyl)-1-methyl-1*H*pyrazol-citrat;
(+)-5-α-[2-(Dimethylamino)ethoxy]benzyl)-1-methyl-1*H*pyrazol;
(-)-5-α-[2-(Dimethylamino)ethoxy]benzyl)-1-methyl-1*H*pyrazol;
(+)-5-α-[2-(Dimethylamino)ethoxy]benzyl)-1-methyl-1*H*pyrazol-citrat;
(-)-5-a-[2-(Dimethylamino)ethoxy]benzyl)-1-methyl-1*H*pyrazol-citrat;
(±)-5-α-[2-(Dimethylamino)ethoxy]-2-thienylmethyl)-1-methyl-1*H*-pyrazol;
(±)-5-α-[2-(Dimethylamino)ethoxy]-2-thienylmethyl)-1-methyl-1*H*-pyrazol-citrat;
(+)-5-α-[2-(Dimethylamino)ethoxy]-2-thienylmethyl)-1-methyl-1*H*-pyrazol;
(-)-5-α-[2-(Dimethylamino)ethoxy]-2-thienylmethyl)-1-methyl-1*H*-pyrazol;
(+)-5-α-[2-(Dimethylamino)ethoxy]-2-thienylmethyl)-1-methyl-1*H*-pyrazol-citrat; und
(-)-5-α-[2-(Dimethylamino)ethoxy]-2-thienylmethyl)-1-methyl-1*H*-pyrazol-citrat;
bei der Herstellung eines Arzneimittels für die Behandlung neurogener Entzündungen bei Säugetieren, einschliesslich beim Menschen.

## Revendications

1. Emploi d'un dérivé d'aryl (ou hétéroaryl) azolilcarbinol dont la formule générale (I) est : 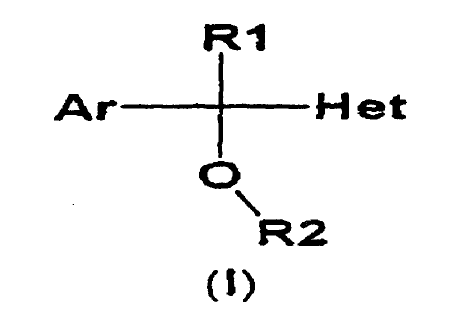 où Ar est un radical phényl ou un radical tiényle, non substitués ou substitués en option par 1, 2 ou 3 substituts égaux ou différents, sélectionnés dans le groupe formé par le fluor, le chlore, le brome, le méthyle, le trifluorométhyle et le métoxy ; R1 est un atome d'hydrogène ou un groupe alquil C₁ - C₄ ; R2 est un radical dialquil (C₁ - C₄) aminoalquile (C₂ - C₃) ou azahétérocyclil-alquile (C₂ - C₃) ; Het est un hétérocycle aromatique azoté à cinq membres qui contient de un à trois atomes d'azote, non substitué ou substitué en option par 1 ou 2 substituts égaux ou différents sélectionnés dans le groupe formé par le fluor, le chlore, le brome et le méthyle ; ou par l'un de leurs sels physiologiquement acceptables, dans l'élaboration d'un médicament pour le traitement de l'inflammation neurogénique chez les mammifères, y compris l'homme.

2. Emploi, selon la revendication 1, d'un composé à formule générale (I), où R1 est sélectionné entre un atome d'hydrogène et le groupe formé par le méthyle, l'éthyle, le propyle, l'isopropyle, le butyle, l'isobutyle, le *sec*-butyle et le *terc*-butyle, dans l'élaboration d'un médicament pour le traitement de l'inflammation neurogénique chez les mammifères, y compris l'homme.

3. Emploi, selon la revendication 1, d'un composé à formule générale (I), où R2 est sélectionné dans le groupe formé par le diméthylaminoéthyle, le diméthylaminopropyle, le diéthylaminoéthyle, le pipéridiéthyle, le morpholinylpropyle et le pyrolidinéthyle, dans l'élaboration d'un médicament pour le traitement de l'inflammation neurogénique chez les mammifères, y compris l'homme.

4. Emploi, selon la revendication 1, d'un composé à formule générale (I), sélectionné dans le groupe formé par :
(±)-5-(α-[2-(dimethylamino)étoxy]benzyle)-1-méthyle-1H-pyrazol ;
Citrate de (±)-5-(α-[2-(dimethylamino)étoxy]benzyle)-1-méthyle-1H-pyrazol ;
(+)-5-(α-[2-(dimethylamino)étoxy]benzyle)-1-méthyle-1H-pyrazol ;
(-)-5-(a-[2-(dimethylamino)étoxy]benzyle)-1-méthyle-1H-pyrazol ;
Citrate de (+)-5-(α-[2-(dimethylamino)étoxy]benzyle)-1-méthyle-1H-pyrazol ;
Citrate de (-)-5-(α-[2-(dimethylamino)étoxy)benzyle)-1-méthyle-1H-pyrazol ;
(±)-5-(α-[2-(dimethylamino)étoxy]-2-tienylméthyle)-1-méthyle-1H-pyrazol ;
Citrate de (±)-5- (α-[2-(dimethylamino)étoxy]-2-tienylméthyle)-1-méthyle-1H-pyrazol ;
(+)-5-(a-[2-(dimethylamino)étoxy]-2-tienylméthyle)-1-méthyle-1H-pyrazol ;
(-)-5-(α- [2- (dimethylamino)étoxy]-2-tienylméthyle)-1-méthyle-1H-pyrazol ;
Citrate de (+)-5-(α-[2-(dimethylamino)étoxy]-2-tienylméthyle)-1-méthyle-1H-pyrazol ;
Citrate de (-)-5-(a-[2-(dimethylamino)étoxy]-2-tienylméthyle)-1-méthyle-1H-pyrazol ;dans l'élaboration d'un médicament pour le traitement de l'inflammation neurogénique chez les mammifères, y compris l'homme.
